(19) Europäisches Patentamt
European Patent Office
Office européen des brevets

(11) Veröffentlichungsnummer: **0 075 613**
**B1**

# (12) EUROPÄISCHE PATENTSCHRIFT

(45) Veröffentlichungstag der Patentschrift: **12.06.85**

(51) Int. Cl.[4]: **C 07 D 295/12**

(21) Anmeldenummer: **81107767.6**

(22) Anmeldetag: **30.09.81**

## (54) Verfahren zur Herstellung von N-(1-Formylamido-2,2,2-trichloräthyl)-morpholin.

(43) Veröffentlichungstag der Anmeldung:
**06.04.83 Patentblatt 83/14**

(45) Bekanntmachung des Hinweises auf die Patenterteilung:
**12.06.85 Patentblatt 85/24**

(84) Benannte Vertragsstaaten:
**AT BE CH FR GB IT LI LU NL SE**

(56) Entgegenhaltungen:
**DE - A - 3 021 579**

**CHEMICAL ABSTRACTS, Band 91, Nr. 15, 8. Oktober 1979, Zusammenfassung 123526n, Seite 590 COLUMBUS, OHIO (US)**
**CHEMICAL ABSTRACTS, Band 55, Nr. 20 2. Oktober 1961, Spalte 19778 COLUMBUS, OHIO (US)**

(73) Patentinhaber: **BASF Aktiengesellschaft, Carl-Bosch-Strasse 38, D-6700 Ludwigshafen (DE)**

(72) Erfinder: **Urbach, Hans, Dr., Alter Kirchenpfad 6, D-6520 Worms (DE)**

**EP 0 075 613 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Unter den bisher beschriebenen Verbindungen der allgemeinen Formel

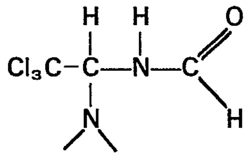

(I)

befinden sich zwei, die fungizide Wirkstoffe sind, nämlich Triforine

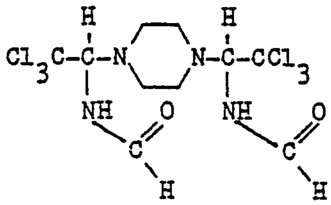

(DE-OS Nr 1901421)
und Trimorfamid

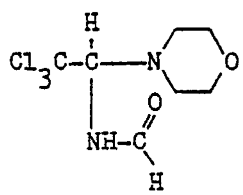

(DE-OS Nr. 2439610).

Die Herstellung von Verbindungen der Formel I hat erstmals F. Feist in „Chem. Ber." 47, 1173 (1914) beschrieben. Eine weitere Reihe von Beispielen lieferte H. Böhme u. a. in „Arch. d. Pharm." 66, 397 (1961).

Beide Autoren verwendeten als Ausgangsmaterial 1,2,2,2-Tetrachloräthylformamid, das in allen Fällen in wasserfreiem Medium mit dem entsprechenden Amin umgesetzt wurde. In weiterer Literatur (DE-OS Nrn. 1901421 und 1946112) wird die Umsetzung vorwiegend in organischem Medium beschrieben.

Es wurde nun gefunden, dass man Trimorfamid mit verbesserter Ausbeute erhält, wenn man Tetrachloräthylformamid mit Morpholin bei Temperaturen von −2 bis etwa 60° C im pH-Bereich 3 bis 14 in einem Zweiphasensystem umsetzt, von dem die eine Phase Wasser ist.

Dieses Ergebnis ist überraschend und war nicht vorauszusehen, weil die erwähnten Autoren das Ausgangsmaterial Tetrachloräthylformamid als eine sehr unbeständige und leicht hydrolisierbare Verbindung beschreiben.

So nennt F. Feist auf S. 117 loc. cit. Tetrachloräthylformamid sehr unbeständig und nach kurzer Zeit vollkommen zersetzt, auf S. 1180 überaus zersetzlich. Auf der gleichen Seite wird erwähnt, dass sich das eine Chloratom (in 1-Stellung) mit N/10 Kalilauge „scharf" titrieren lässt, was für den Fachmann bedeutet, dass es leicht hydrolysierbar ist. H. Böhme berichtet auf S. 307 loc. cit., dass Tetrachloräthylformamid durch das reaktionsfähige Chloratom in α-Stellung charakterisiert ist, welches leicht der Hydrolyse unterliegt. Auch die homologe Verbindung mit einer $CH_2$-Gruppe mehr im Molekül, das 1,2,2,3-Tetrachlorpropylformamid der nachstehenden Formel

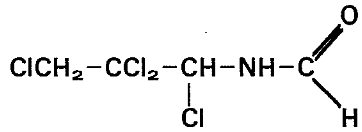

wird im „Journ. f. prakt. Chemie" Bd. 316, S. 63 (1974) von H. Zimmer u. a. beschrieben, und zwar soll das Chlor in 1-Stellung sehr reaktionsfähig und besonders empfindlich gegen Wasser sein, so dass z. B. die Luftfeuchtigkeit ausreicht, um eine Hydrolyse zu verursachen.

Bei dieser Empfindlichkeit des Tetrachloräthylformamids gegenüber Wasser war es nicht zu erwarten, dass die Umsetzung mit Morpholin in einem Zweiphasensystem ohne merkliche Hydrolyse möglich sein würde und dabei die Ausbeute gegenüber Literaturangaben noch erheblich gesteigert wird, mit 96 bis 97% d. Th. praktisch quantitativ. Zudem ist die Reinheit der so erhaltenen Verbindungen sehr noch.

Als organische Phase in dem Zweiphasensystem eignen sich praktisch alle Lösungsmittel, die in Wasser nicht oder nur wenig löslich sind und die eine gewisse Löslichkeit für Tetrachloräthylformamid besitzen, z. B. aromatische Kohlenwasserstoffe und chlorierte aromatische Kohlenwasserstoffe, wie Benzol, Toluol, Xylol, Cumol, Chlorbenzol, Dichlorbenzol, oder Nitrobenzol, ausserdem aliphatische Verbindungen, wie Methylenchlorid, Chloroform, Tetrachlorkohlenstoff, Essigester, Isobutylacetat, Diäthyläther, Anisol, Acetophenon, Cyclohexan. Auch Gemische solcher Lösungsmittel können verwendet werden.

Die Reaktionstemperatur liegt zwischen −2 und etwa 60, vorteilhaft zwischen 0 und 30, bevorzugt zwischen 20 und 25° C. Da die Reaktion exotherm ist, ist es zweckmässig, durch Kühlung für die Einhaltung der vorgesehenen Temperatur zu sorgen.

Vorteilhaft innerhalb des gekennzeichneten pH-Bereiches von 3 bis 14 ist der neutrale bis alkalische pH-Bereich, insbesondere der zwischen 8 und 10, bevorzugt zwischen 8,5 und 9. Wenn man das Morpholin vorlegt, genügt es, den pH-Bereich gewünschtenfalls erst zu regeln, wenn etwa die Hälfte des Morpholins durch Umsetzung verbraucht ist. Bis dahin stellt sich der pH-Wert durch das freie Morpholin ein. Zur Regelung kann man alkalische Verbindungen benutzen, z. B. Natronlauge, Natriumcarbonat, Natriumacetat, Kalilauge, Kaliumcarbonat. Es ist zweckmässig, auf diese Weise den pH-Bereich im Verlauf der Umsetzung auf höher als pH 8 einzustellen und zu halten.

Für eine wirtschaftliche Ausnutzung der Reaktionspartner ist es zweckmässig, das Molverhältnis von Tetrachloräthylformamid: Morpholin zwischen 0,5:1 und 2:1 zu wählen, vorteilhaft zwischen 0,8:1 und 1,2:1, bevorzugt bei 0,95:1 bis 0,98:1.

Das Tetrachloräthylformamid kann dem Reaktionsansatz in fester Form als Suspension oder, wegen der leichteren Handhabung, als Lösung zugesetzt werden. Wenn man ein Lösungsmittel verwendet, in dem sich Tetrachloräthylformamid schwer oder nicht vollständig löst, kann es vorteilhaft sein, die Lösung auf erhöhter Temperatur zu halten.

Es ist nicht erforderlich, reine Ausgangsstoffe zu benutzen. Man kann auch z. B. ein ungereinigtes Tetrachloräthylformamid einsetzen, das man durch Chlorierung von Chloralformamid mit Thionylchlorid erhalten hat, ohne dass Ausbeute oder Reinheit der erhaltenen Verbindungen merklich sinken.

Die Ausbeute liegt normalerweise bei 90%, kann jedoch mit Hilfe eines Phasentransferkatalysators bis auf 97% gesteigert werden. Geeignete Katalysatoren sind die gebräuchlichen Oniumverbindungen, bevorzugt quaternäre Ammoniumverbindungen, wie Tetraäthylammoniumbromid, Triäthylbenzylammoniumchlorid, Tetrabutylammoniumhydrogensulfat. Man verwendet sie im allgemeinen in üblichen Mengen, nämlich 0,1 bis 50 Mol-%, bezogen auf Morpholin.

Die Reaktionszeit ist verhältnismässig kurz, da die Umsetzung glatt und rasch verläuft. Sie liegt im Bereich vom 5 bis 180, im allgemeinen bei 30 min. Das Ende der Umsetzung kann man leicht daran erkennen, dass der pH-Wert im gewünschten Bereich ohne Zugabe weiterer Base konstant bleibt.

Das Verfahren dieser Verbindung kann z. B. folgendermassen ausgeführt werden:

Morpholin, ggf. ein Teil des Lösungsmittels und Wasser, ggf. zusätzlich ein Phasentransferkatalysator, werden vorgelegt und das Tetrachloräthylformamid in fester Form, als Suspension in einem Lösungsmittel oder gelöst in einem Lösungsmittel zugegeben. Durch Zugabe einer Base, am wirtschaftlichsten Natronlauge, wird der pH-Wert während der Reaktion im leicht alkalischen Bereich gehalten, wobei der pH-Wert am einfachsten mit einer pH-Elektrode kontrolliert werden kann. Nach dem Ende der Reaktion werden bei Verwendung eines Lösungsmittels, das Trimorfamid unter den Reaktionsbedingungen vollständig löst, die Phasen getrennt und das Lösungsmittel durch Destillation von Trimorfamid abgetrennt. Bei Verwendung eines Lösungsmittels, welches das entstandene Trimorfamid nur teilweise gelöst hat, wird die entstandene Suspension mit einer der üblichen Isolierungsvorrichtungen, wie Filter oder Schleuder, in Feststoff und Filtrat getrennt. Im Filtrat werden die Phasen getrennt, und die organische Phase wird ggf. ebenfalls zur Isolierung aufgearbeitet. Eine zusätzliche Extraktion der wässerigen Phase erübrigt sich in der Regel, da Trimorfamid in Wasser sehr schwer löslich ist.

In einer bevorzugten Form des Verfahrens, die besonders wirtschaftlich ist, rührt man zunächst Chloral und Formamid mehrere Stunden in Toluol bei erhöhter Temperatur und setzt ohne Isolierung des entstandenen Chloralformamids Thionylchlorid zu. Man rührt wieder einige Stunden bei erhöhter Temperatur und erhitzt zur Entfernung von überschüssigem Thionylchlorid die Reaktionslösung unter vermindertem Druck, bis etwa 1/20 des Volumens abdestilliert ist. Die so erhaltene Lösung lässt man in eine wässerige Lösung von Morpholin einlaufen, wobei durch Zugabe von Natronlauge dafür gesorgt wird, dass der anfangs bei 11,4 gelegene pH-Wert nicht unter 8,5 fällt. Man erhält so mit hoher Wirtschaftlichkeit Trimorfamid mit einer Gesamtausbeute, bezogen auf eingesetztes Formamid, von 88%.

In den folgenden Beispielen verhalten sich Volumenteile zu Gewichtsteilen wie Liter zu Kilogramm.

*Beispiel 1*

In einen Rührbehälter, der mit einer pH-Elektrode ausgerüstet ist, werden

90 Gew.-Teile Morpholin,
3 Gew.-Teile Tetraäthylammoniumbromid,
50 Vol.-Teile Toluol und
1000 Vol.-Teile Wasser vorgelegt.

Aus einer Vorlage lässt man im Laufe von 30 min eine auf 50° C gehaltene Lösung von

211 Gew.-Teilen Tetrachloräthylformamid in
350 Vol.-Teilen Toluol zulaufen,

wobei man durch Kühlung die Temperatur bei 20 bis 25° C und durch Zugabe von Natronlauge (25%ig) den pH-Wert bei 8,5 hält.

Nach 30 min Nachrührzeit saugt man die erhaltene Suspension ab, wäscht mit Wasser chloridfrei und erhält nach dem Trocknen 254 Gew.-Teile Trimorfamid, das dünnschichtchromatographisch einheitlich ist und einen Schmelzpunkt von 113,5° C besitzt. Das entspricht einer Ausbeute von 97% d. Th., bezogen auf Tetrachloräthylformamid.

*Beispiel 2*

Führt man die Umsetzung von Beispiel 1 ohne den Zusatz von Phasentransferkatalysatoren aus, beträgt die Ausbeute 88%.

## Patentansprüche

1. Verfahren zur Herstellung von N-(1-Formylamido-2,2,2-trichloräthyl)morpholin durch Umsetzung von 1,2,2,2-Tetrachloräthylformamid mit Morpholin bei Temperaturen von −2 bis etwa 60° C im pH-Bereich 3 bis 14 in einem Zweiphasensystem, von dem die eine Phase Wasser ist, dadurch gekennzeichnet, dass man während der Umsetzung den pH-Bereich durch Zugabe alkalischer Verbindungen auf höher pH 7 einstellt.

2. Verfahren nach Anspruch 1, dadurch gekennzeichnet, dass man bei der Umsetzung einen Phasentransferkatalysator benutzt.

3. Verfahren nach einem der Ansprüchen 1 oder 2, dadurch gekennzeichnet, dass man die Umsetzung mit einem 1,2,2,2-Tetrachloräthylformamid durchführt, das ohne Isolierung der Zwischenstufen aus Chloral und Formamid hergestellt wurde.

## Claims

1. A process for the preparation of N-(1-formylamido-2,2,2-trichloroethyl)morpholine by reacting 1,2,2,2-tetrachloroethylformamide with morpholine at a temperature of from −2 to about

60° C, at a pH of from 3 to 14, in a two-phase system of which one phase is water, wherein the pH is kept at above 7 during the reaction by adding an alkaline compound.

2. A process as claimed in Claim 1, wherein a phase transfer catalyst is used in the reaction.

3. A process as claimed in Claim 1 or 2, wherein the reaction is carried out with 1,2,2,2-tetrachloroethylformamide which has been prepared from chloral and formamide without isolating the intermediates.

## Revendications

1. Procédé de préparation de N-(1-formylamido-2,2,2-trichloréthyl)morpholine par réaction de 1,2,2,2-tétrachloréthylformamide avec de la morpholine, à des températures de −2 à environ 60° C, dans une zone de pH de 3 à 14, dans un système à deux phases, dont l'une des phases est de l'eau, caractérisé par le fait que, pendant la réaction, on règle la zone de pH, par addition de composés alcalins, au-dessus de pH 7.

2. Procédé selon la revendication 1, caractérisé par le fait que, pour la réaction, on utilise un catalyseur de transfert de phase.

3. Procédé selon l'une des revendications 1 ou 2, caractérisé par le fait que l'on effectue la réaction avec un 1,2,2,2-tétrachloréthylformamide qui, sans isolement de l'étape intermédiaire, a été préparé à partir de chloral et de formamide.